# EUROPEAN PATENT APPLICATION

(11) **EP 1 739 405 A1**
(43) Date of publication of application: **03.01.2007**
(21) Application number: 06253382.3
(22) Date of filing: 28.06.2006
(51) Int. Cl.: G01N 15/02, F24F 3/16, B01D 46/46, A61L 9/20

(54) **Method and apparatus for detecting and removing airborne microbes**

(30) Priority: 28.06.2005 JP 2005187797
(71) Applicant: Hirayama Setsubi Kabushiki Kaisha, Yamato-shi, Kanagawa-ken (JP)
(72) Inventor: Hirayama, Kensuke, Yamato-shi Kanagawa-ken (JP)
(74) Representative: Ellis, Katherine Elizabeth Sleigh

(57) **Abstract**

A method and an apparatus for detoxifying harmful objects such as microbes, their toxins, and pathogenic viruses such as those of severe acute respiratory syndrome (SARS) by means of a photo-catalyst technique and an air cleaning technique so as to eliminate or minimize the damage due to such harmful objects. An air cleaner (12) incorporating air filters (16,17,19) is placed in a corner or suitably adjacent to a wall in an inner chamber (10). A dust gauging sensor (20) is mounted on or in proximity to a side wall of the air cleaner (12) to detect the amount of dust inclusive virus particles based on each airborne particle diameter circulating in a building space while a computer (23) for processing the output of the dust gauging sensor (20) is placed in an inner chamber or outside of the chamber. When the number of airborne microbe particles having a predetermined particle diameter exceeds a predetermined number in a unit of time, the air filter (17) is activated by a signal from the sensor (20) to annihilate the viruses.

## Description

This invention relates to a method and an apparatus for detecting and removing airborne microbes in a building space.

Countries all over the world are forced to take urgent countermeasures against the recent appearance of novel infectious diseases caused by pathogens such as SARS corona virus. More specifically, countermeasures against attacks of microbes such as anthrax bacilli and botulinus bacilli, toxins of such microbes, and viruses such as smallpox virus or stealth virus as well as severe acute respiratory syndrome (SARS) are urgently required. Such measures including techniques for removing harmful substances contained in gaseous phases by means of filters designed to decompose organic gaseous substances and microbes by irradiation of light (JP 2002-355299, page 2, right column, line 42 - page 3, left column, line 3) and techniques for leading air in a chamber into an air cleaner to remove dust and subsequently ionizing the air by means of an ionization unit and discharging electrode wires so as to allow the anode plate to adsorb pathogenic microbes and harmful chemical substances (JP 2002-136585, page 4, right column, line 34 - page 5, left column, line 17, and FIG. 2) are well known. Additionally, a technique for annexing a harmful microbe detection apparatus and/or a particle concentration gauging apparatus to an air conditioning system so as to remove microbes such as anthrax bacilli and tubercle bacilli and viruses such as smallpox viruses by means of a filter and to analyze the samples taken from an inlet or an outlet of the filter is also known (JP 2003-202129, page 3, left column, line 40 - page 3, right column, line 6, and FIG. 2).

Severe acute respiratory syndrome (SARS) refers to an infectious disease caused by SARS corona virus that operates as a pathogen. A person can be infected with this disease when exposed to a coughing patent or when in direct contact with a splash of saliva or some other bodily liquid produced from a patient. Symptoms appear after an incubation period of two to seven days. A patient infected with SARS is given a treatment using anti-infection equipment including a mask and a gown and held in a compact negative pressure chamber in such a manner as to isolate him/her from other patients by means of partitions in a treatment facility. Treatment includes controlling of a patient's entire body and respiratory system, and alleviating of his/her symptoms. However, because of the fact that prevention against this disease merely relies on hand-washing and gargling, research and development not only for quick diagnostic methods and treatment for SARS but also for anti-virus agents and preventive vaccines are a top priority issue in the field of medical technologies.

Preventing particulate, liquid or gaseous harmful substances from spreading and preventing microbes, toxins and pathogenic viruses from becoming airborne and spreading are a problem to be solved in all the countries of the world. Thus, the object of the present invention is to provide a method and an apparatus adapted to raise the processing rate of an air filter or each of the air filters in an air cleaner and to sterilize inside of a building when dust containing pathogenic microbes having a particle diameter greater than a predetermined threshold value is detected in the building so as to reduce or control the number of sufferers (patients including those lethally afflicted).

According to a first aspect of the present invention, there is provided a method of detecting and removing floating microbes as specified in claim 1.

According to a second aspect of the present invention, there is provided a method of detecting and removing floating microbes as specified in claim 2.

According to a third aspect of the present invention, there is provided an apparatus for detecting and removing floating microbes in a building as specified in claim 3.

In one aspect of the present invention, the above object is achieved by providing a method of detecting and removing floating microbes, said method comprising: detecting the number of particles of dust based on each particle diameter floating in a building by means of a dust gauging sensor; outputting data indicating an abnormal increase in the number of particles of a predetermined particle diameter to a computer for processing the data about the abnormal increase when such an abnormal increase is detected in the number of the particles of a predetermined particle diameter beyond a predetermined value in a unit of time, and recording the outcome of the processing operation of the computer in the form of a numerical table or graph for confirmation.

In another aspect of the present invention, there is provided a method of detecting and removing floating microbes, said method comprising: arranging an air cleaner main body incorporating one or more air filters and a dust gauging sensor in a building; detecting the number of particles of dust based on each particle diameter floating in a building by means of the sensor; inputting data indicating an abnormal increase in the number of particles of a predetermined particle diameter in dust to a computer for processing when such an abnormal increase is detected in the number of particles having a predetermined particle diameter beyond a predetermined value in a unit of time, and subsequently raising the flow rate of air to be processed by the air cleaner so as to remove the increased amount of particles beyond the predetermined value according to the output of the computer.

In still another aspect of the present invention, there is provided an apparatus for detecting and removing floating microbes inside a building, said apparatus comprising: an air cleaner main body incorporating one or more air filters and a dust sensor for counting the number of particles of dust based on each particle diameter floating in air, both provided inside of a building, and a computer provided inside or outside of a chamber space for processing the output of the dust gauging sensor; wherein when the sensor detects an abnormal increase in the number of dust particles having a predetermined particle diameter beyond a predetermined value in a unit of time, such abnormally increased particles are removed by raising the flow rate of air to be processed by the air cleaner according to instructions of the computer. Preferably, such an air cleaner is modularized so that a plurality of air cleaners may be placed depending on the internal capacity of the building.

Thus, according to the present invention, an air cleaner main body incorporating one or more air filters and a dust sensor are placed in a building for setting a predetermined value by detecting the number of particles of dust based on each particle diameter floating in air so that when an abnormal increase is detected in the number of particles having a predetermined particle diameter beyond a predetermined value in a unit of time, data of such an abnormal increase is output to a computer for processing the data so as to remove the increased amount of particles beyond the predetermined value. Therefore, abnormally increased particles within a building, such as particles contained viruses, floating microbes and biological agents, can be removed by one or more air filters provided within the air cleaner.

Preferred embodiments of the present invention are described below, by way of example only, with reference to the accompanying drawings in which:
FIG. 1 is a schematic front view illustrating the first embodiment of an apparatus for detecting and removing airborne microbes to be used with a method of detecting and removing airborne microbes of the present invention;
FIG. 2 is a schematic perspective view illustrating the apparatus of FIG. 1;
FIG. 3 is a schematic perspective view illustrating the second embodiment of the apparatus of the present invention;
FIG. 4 is a schematic perspective view illustrating the third embodiment of the apparatus of the present invention;
FIG. 5 is an enlarged schematic front view illustrating a mounting condition of a dust gauging sensor mounted on the ceiling of a building;
FIG. 6 is an enlarged schematic front view illustrating a mounting condition of a dust gauging sensor hidden in the ceiling of a building;
FIG. 7 is an enlarged schematic front view illustrating a mounting condition of dust gauging sensor provided in proximity to an air blowing port of an air circulation duct system; and
FIG. 8 is a schematic perspective view illustrating the fourth embodiment of the apparatus of the present invention.

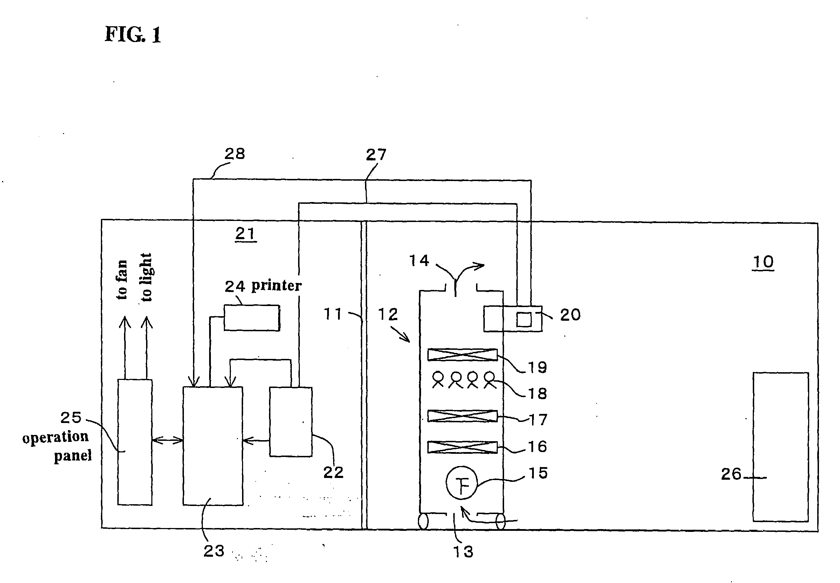

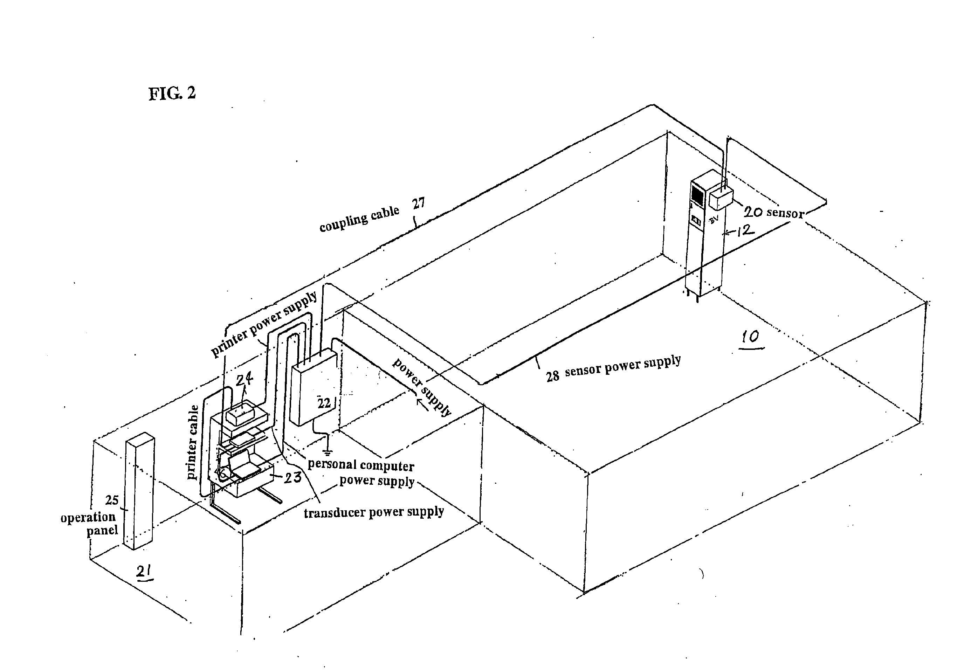

FIG. 1 is a schematic front view of the first embodiment of an apparatus for detecting and removing floating microbes according to the present invention and FIG. 2 is a schematic perspective view of the apparatus of FIG. 1.

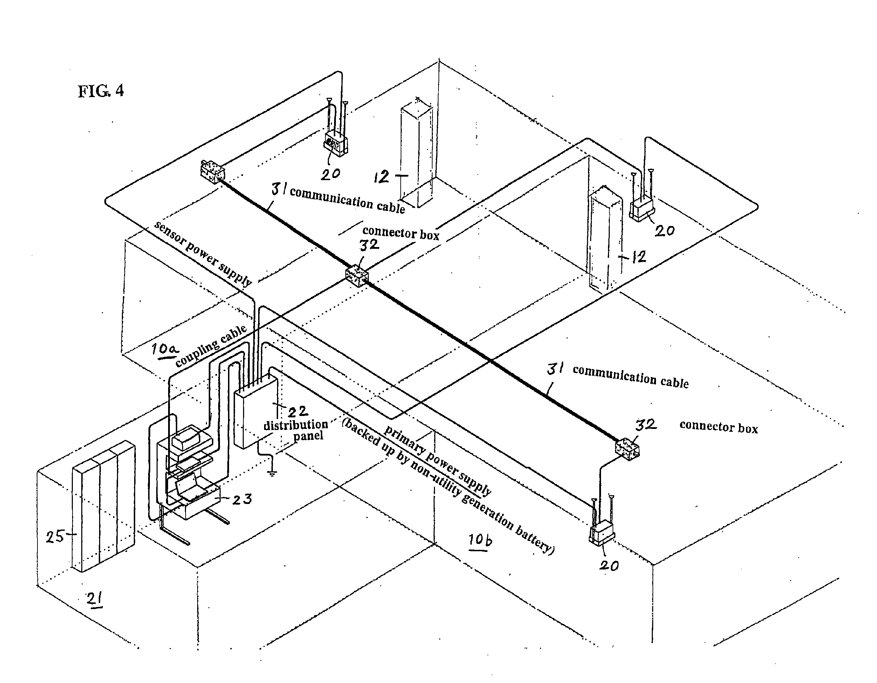

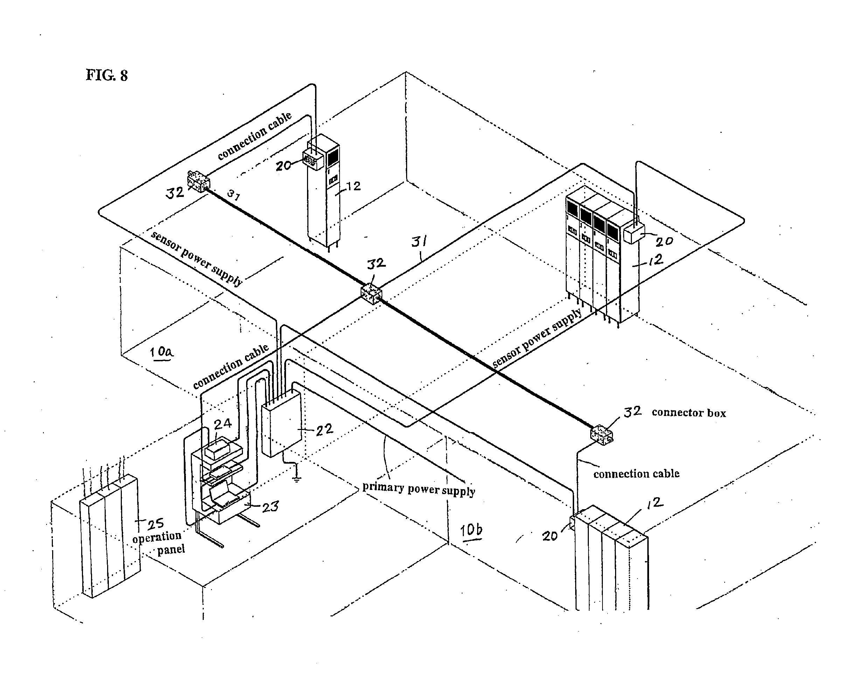

As shown in FIGS. 1 and 2, an air cleaner 12 that incorporates air filters 16, 17, 18 is placed at a corner or suitably adjacent to a wall in an inner chamber (room). A dust gauging sensor 20 is mounted on or in proximity to a side wall of the air cleaner 12 to detect the amount of airborne dust-inclusive virus particles based on each particle diameter circulating in a building space while a computer 23 for processing the output of the dust gauging sensor 20 is placed in an inner chamber or outside of the chamber. When the number of airborne microbe particles in the circulating air and having a predetermined particle diameter exceeds a predetermined number in a unit of time, the air filter 17 is activated by a signal from the sensor 20 to annihilate the viruses. As shown in FIGS. 4 and 8, the air cleaner 12 is modularized and more than one air cleaner may be placed depending on the volume of the building space.

An air cleaning apparatus 12 has an air suction port 13 in the lower part and an air blowing port 14 in the upper part thereof, respectively. A dust detection sensor 20 is mounted on a side wall of the air cleaner 12 in proximity to an air blowing port 14. A distribution panel 22, a computer 23, and a control panel 24 are provided at an operation room 21 separated from a room 10 by a partition 11. A coarse dust removing filter 1, a high performance filter 17, an ultraviolet ray light 20, and an intermediate performance filter 19 are housed within the air cleaning apparatus 12 in such a manner to be placed above the air suction port 13. An imperceptible amount of airborne particles are continuously measured by the sensor 20 so that when the sensor 20 detects an abnormal increase in the amount of particles, the sensor 20 transmits a signal to a computer 23 so as to advise through an alarm and a display on the monitor screen of the computer (not shown) placed in, for example, an operation room or a corresponding room 10. In FIGS. 1 and 2, reference numeral 26 denotes a door and reference numerals 27 and 28 denote a coupling cable.

An air particle counter of a semiconductor laser light source side light scattering type (e.g., TRANS TECHNO Co., Ltd.) is used as a dust gauging sensor 20 to detect particles having a minimal particle diameter of 0.3 or 0.5µm. In the measuring process, a particle diameter of airborne microbes (pathogens such as viruses, toxins and rickettsias) is specified, for instance, within a range of 1.0 to 10.0µm as described below. Then, these specified type particles are selected to specify a reference value for determining an abnormal increase. In order to establish a distinguishing method between an abnormal increase and electronic noise, an abnormal increase is determined at least once every air ventilation period. Accumulation and analysis of measuring data, and indications, instructions, or the like are performed with a personal computer using dedicated computer software.

According to the present invention, when cleaning the air introduced into the chamber, attributes such as particle diameter and amount/numbers of true fungi, microbes, spores of such germs, or pathogenic viruses including those of severe acute respiratory syndrome (SARS) are detected and intoxicated by photo-catalytic technology and air cleaning technology so as to eliminate or minimize damage caused by those fungi. When detecting microbes (viruses, rickettsias, fungi and spores) and cleaning the air in a chamber by means of an air cleaner, a dust gauging sensor is provided at a desired position in the chamber or a side wall of an air cleaner main body so as to remove airborne particles of dust having a particle diameter of not smaller than 0.3µm by not less than 99.997% (0.003% leak). Approximately 1,000 viruses can exist in 1µm when a high performance filter is used.

When an abnormal increase is detected in the number of particles of dust, a buzzer alarm sounds and an alarm image is displayed on a monitor screen of a computer, and the air cleaner intensifies its operation. As a result, the hosts of viruses are eliminated and no living cells would enter the chamber. Additionally, particles of dust sucked and removed through a neutral filter are irradiated by ultraviolet rays for one second so as to damage viruses. The leaked particles are sterilized and inactivated, or damaged on the surface and partially disintegrated by the irradiation of ultraviolet rays.

Then, a graph is prepared by using a computer program to indicate the relationship between the number of floating particles (e.g., two phases: one for particles with a diameter not smaller than 1µm and the other for particles with a diameter not less than 5µm) which is a longitudinal axis, and time, which is a horizontal axis. Detection of three to five abnormal increases in a unit of time is considered as an emergency situation while detection of two or less abnormal increases in a unit of time is not regarded as an emergency situation.

Unique definitions: For example, two diameters are defined for particles including one for particles having a diameter not less than 1µm and the other for particles having a diameter not less than 5µm. Particles of a diameter not less than 1µm fall naturally but those of a diameter not less than 5µm become airborne and move around in a static space. A dust gauging sensor may be operated to detect particles several times to prevent operation errors but five times may be standard. However, even one detection can generate an alarm when an abnormal increase is detected. For example, assuming that 1cc (cubic c centimeter) of airborne dust exists in a building with a capacity of 3,000m³, particles in a range of 0.3 - 10µm or up to 20µm can be detected by means of light reflex in which an alarm is generated at the peak of an abnormal increase.

### Principle ofAutomatically Controlled Dust Gauging Operation

Many particles having a diameter of less than 1µm tend to coagulate naturally to grow where coagulated particles naturally fall in a static space but float in a dynamic space. A particle having a diameter of 1µm that floats in a slight air flow (of 0.0006m/sec) is used as an index for particles having a diameter of smaller than 1µm. Thus, the spread of scattering particulate substances can be quickly detected by measuring an abnormal increase of particles of these two diameters. Furthermore, artificially produced particles such as those of albumen for incubating viruses (1µm) and spores of anthrax bacilli (1.5 to 3µm) become visible when they naturally coagulate to have a diameter not smaller than 10µm. Then, they tend to fall and cannot easily remain airborne. Therefore, a particle that has grown to have a diameter of 5µm, which is substantially the upper limit of the diameter of particles airborne in an ordinary room, is used as an index for particles with a diameter of not less than 1µm (can float in an air flow of 0.015m/sec or higher). Additionally, because particles having a diameter of 5µm are caught by a filter as airborne dust and do not circulate, only those produced and/or scattered in a room will be measured, which enables an abnormal increase to be detected easily.

The following measures can be further taken:
a) Changes in the diameter and the amount of floating particles are continuously measured over time with a dust gauging sensor deployed in the chamber and the occurrence of an abnormal increase is notified by an alarm (sound) and a light (light) of a computer or a monitor panel.
b) An abnormal increase is generally determined within a single ventilation period.
c) Accumulation and analysis of measuring data, and indications, instructions, or the like are performed with a personal computer using dedicated computer software.
d) A technique for distinguishing an abnormal increase from noise is established. Representative particles are selected and a reference quantity for determining an abnormal increase is specified.

The amount of floating particles having a diameter not smaller than 1µm and those (or an airborne particle sensor) on-line. An alarm is generated and a signal is output through a computer when an abnormal increase beyond a specified value is detected. The sensor can compute the average value, the largest value, the smallest value, the standard deviation and the totaled data for any arbitrarily selected period and re-total the collected data (e.g., every ten minutes or so, or every several hours). A specified numerical value may be obtained in such a manner that an average value of an index particle (for each ventilation time) detected for the preceding five weeks until one day before and a value measured at the same time of the day are compared with each other so that an abnormal increase may be detected when the measured value is/exceeds 10 times more than the average value to generate an alarm and a signal. The average value is recalculated and updated once a day by computer processing. With this arrangement, it is possible to immediately discover an anomaly and to identify the location of occurrence. Subsequently, emergency evacuation, sealing of the location, or cleaning of air by activating an air cleaner can be promptly performed. Additionally, detection and alert of gaseous substances can be made in a similar manner.

When an abnormal increase is detected during an operation,
(1) an air cleaner comprising individual air filters is activated, in which the air cleaner and the air filters can be manually operated in an emergency situation where, for example, influenza prevails;
(2) the air is forced through the air filters and the air cleaner without bypassing, in which the air cleaner and the air filters can be manually operated in an emergency situation where, for example, influenza prevails;
(3) the flow rate of the air through the centralized type air filters and the air cleaner is raised and, if the air flow rate is reduced, they are bypassed. With this arrangement, the amount of index particles in a room is maintained at a constant level under ordinary circumstances.

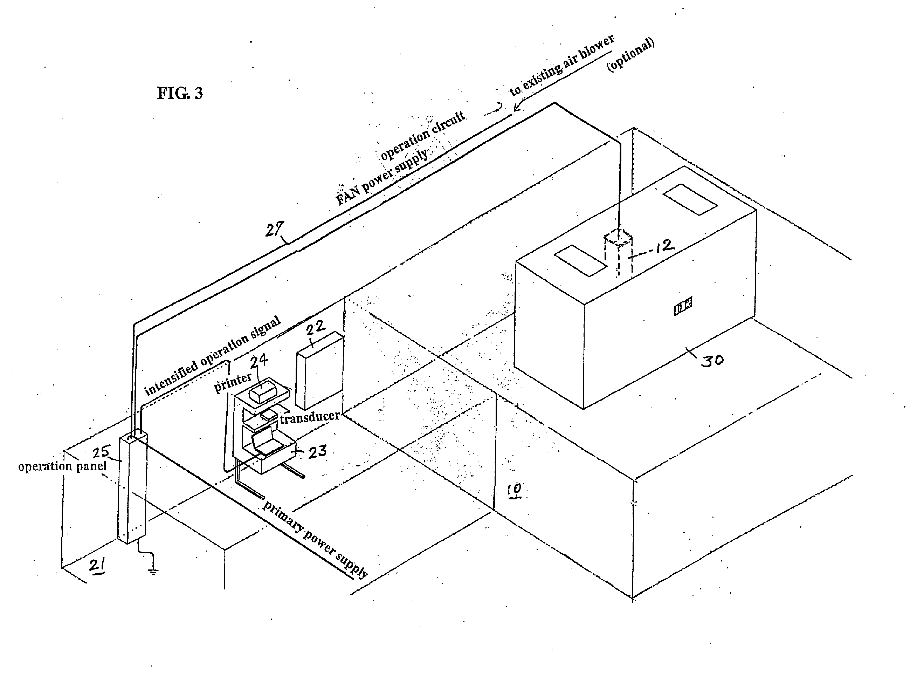

FIG. 3 is a schematic perspective view illustrating a second embodiment of an apparatus of the present invention. Referring to FIG. 3, a hermetically sealed chamber 30 is placed in an inner chamber space (room) 10 and an air cleaner 12 equipped with a dust gauging sensor 20 is provided inside of the chamber 30. As shown in FIG. 2, a distribution panel 22, a computer 23 and an operation panel 24 are placed within an operation room 21 that is separated from the room 10 by a partition 11. An imperceptible amount of floating particles in the hermetically sealed chamber 30 is continuously measured by the sensor 20 and if an abnormal increase in the amount of particles is detected, a signal is transmitted to the computer 23 so as to notify with an alarm and a light (not shown).

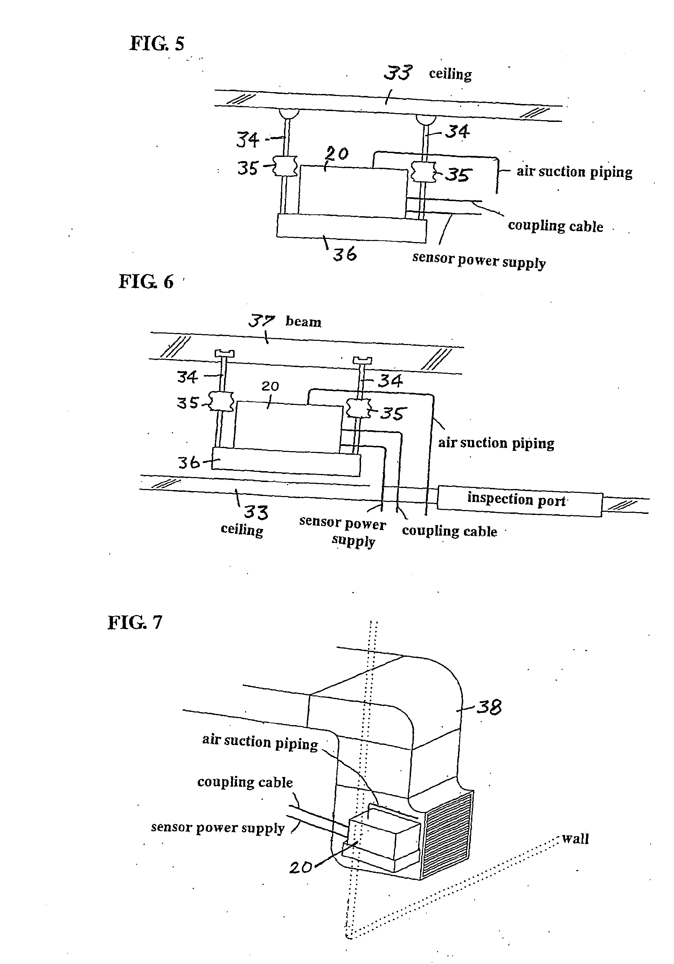

FIG. 4 is a schematic perspective view illustrating a third embodiment of an apparatus of the present invention. As shown in FIG. 4, an air cleaner 12 is deployed in corners in a plurality of inner chamber spaces (rooms) 10a, 10b, respectively, and a dust gauging sensor 20 is mounted on the ceiling of the rooms which is separated from an air blowing port of the air cleaner 12 by an appropriate distance. Connector boxes 32 are provided at communication cables 31 introduced from a computer 23 to connect with a sensor 20 located in each room. An imperceptible amount of airborne particles is continuously measured by the sensor 20 and if an abnormal increase in the amount of particles is detected, a signal is transmitted to the computer 23 so as to notify with an alarm and a light as explained previously. As shown, for example in FIG. 5, each of the dust gauging sensors 20 exposed from the ceiling may be mounted in such a manner firmly to fix suspended metal fixtures 34 having an earthquake-proof device 35 to the ceiling 33, on which a horizontal plate 36 is mounted. The dust gauging sensors 20 may not be exposed from the ceiling but alternatively mounted under a beam 34 in the ceiling as shown in FIG. 6. Furthermore, they may be mounted inside of a side wall of an air circulation duct 38 as shown in FIG. 7.

FIG. 8 is a schematic perspective view illustrating a fourth embodiment of an apparatus of the present invention. An air cleaner 12 is deployed in corners of a plurality of inter chamber spaces (rooms), respectively. More specifically, an air cleaner 12 is deployed in the first inner chamber space (room) 10a, while a plurality of air cleansers 12, 12 are placed at a diagonal corners of the second inner chamber space 10b, the number of said air cleaners placed at each corner depending on the capacity of the space 10b. A dust gauging sensor 20 is deployed in proximity to each group of air cleaners 12. Each of the sensors 20 is connected to a communication cable 31 introduced from a computer 23. An imperceptible amount of floating particles is continuously measured by the sensor 20 and if an abnormal increase in the amount of particles is detected, a signal is transmitted to the computer 23 so as to notify with an alarm and a light as explained previously.

As described above, according to preferred embodiments of the invention, the number of particles of each particle diameter of airborne dust inclusive virus particles is detected by a dust gauging sensor placed in proximity to an air blowing port of an air cleaner housing one or more air filters or on an inner wall of an air circulation duct, so that when the number of virus particles of a predetermined particle diameter exceeds a predetermined amount, the air filter (or filters) in the air cleaner is activated to annihilate viruses.

The disclosures in Japanese patent application No. 2005-187797 from which this application claims priority, and in the abstract accompanying this application are incorporated herein by reference.

## Claims

1. A method of detecting and removing floating microbes, said method comprising:
detecting the number of particles of dust based on each particle diameter airborne in a building by means of a dust gauging sensor;
outputting data indicating an abnormal increase in the number of particles of a predetermined particle diameter to a computer for processing the data about the abnormal increase when such an abnormal increase is detected in the number of the particles of a predetermined particle diameter beyond a predetermined value in a unit of time, and
recording the outcome of the processing operation of the computer in the form of a numerical table or graph for confirmation.

2. A method of detecting and removing floating microbes, said method comprising:
deploying an air cleaner main body incorporating one or more air filters and a dust gauging sensor in a building;
detecting the number of particles of dust based on each airborne particle diameter in a building by means of the sensor;
inputting data indicating an abnormal increase in the number of particles of a predetermined particle diameter in dust to a computer for processing when such an abnormal increase is detected in the number of particles having a predetermined particle diameter beyond a predetermined value in a unit of time, and
subsequently raising the flow rate of air to be processed by the air cleaner so as to remove the increased amount of particles beyond the predetermined value according to the output of the computer.

3. An apparatus for detecting and removing floating microbes in a building, said apparatus comprising:
an air cleaner main body (12) incorporating one or more air filters (16, 17, 18) and a dust sensor (20) for counting the number of particles of dust based on each airborne particle diameter, both provided inside of a building, and a computer (23) provided inside or outside of a chamber space for processing the output of the dust gauging sensor; wherein when the sensor detects an abnormal increase in the number of dust particles having a predetermined particle diameter beyond a predetermined value in a unit of time, such abnormally increased particles are removed by raising the flow rate of air to be processed by the air cleaner according to the instructions of the computer.

4. An apparatus as claimed in claim 3, wherein said air cleaner is modularized so that a plurality of air cleaners may be deployed depending on the internal capacity of the building.
